# EUROPEAN PATENT APPLICATION

(11) **EP 2 111 790 A1**
(43) Date of publication of application: **28.10.2009**
(21) Application number: 08103668.3
(22) Date of filing: 23.04.2008
(51) Int. Cl.: A61B 5/087, A61B 5/091, A61M 16/00

(54) **Measuring system, computer program, set of a measuring device and a computer program and method for operating the measuring system**

(71) Applicant: Microlife Intellectual Property GmbH, 9443 Widnau (CH)
(72) Inventor: Lin, Kin-Yuan, 114, Taipei (TW); Peng, Hsieng Chin, 310, Hsinchu County (TW)
(74) Representative: Hepp, Dieter

(57) **Abstract**

The present invention relates to a measuring system (2) for electronically measuring the volume flow expired and/or inspired by a user, to a computer program, to a set of a measuring device and a computer program and a method for operating the measuring system (2).

The measuring system (2) comprises a measuring unit (7) for continuously monitoring the volume flow of air when the user is expiring and/or inspiring, a first memory section (9) for storing data of a reference expiration and/or inspiration volume flow profile (21) and at least a second memory section (10) for storing data of a measured expiration and/or inspiration volume flow profile (22a, 22b). The system (2) further comprises display means (3) for displaying flow profiles (19),
wherein the measuring system is construed for displaying a representation of the reference flow profile (21) and the measured flow profile (22a, 22b), which allows a comparison of the profiles (19).

## Description

The present invention relates to a measuring system for electronically measuring the volume flow expired and/or inspired by a user, to a computer program, to a set of a measuring device and a computer program and a method for operating the measuring system according to the preambles of the independent claims.

Measuring systems for electronically or mechanically measuring the volume expired and/or inspired by a user, particularly peak flow meters have been around for a number of years. Many clinicians recognize that daily Peak Expiratory Flow (PEF) measurements can provide early warnings of an asthma attack.

The peak flow meter measures Peak Expiratory Flow (PEF), defined as the maximum rate at which an individual can expel air from the lungs, using maximal effort from full inhalation. PEF usually is measured in litre per second or per minute.

A peak flow meter, for example a personal spirometer, typically measures further respiratory parameters, including the Forced Expiratory Volume (FEV1), defined as the volume of air expelled by an individual in the first second of exhalation, using maximal effort from full inhalation. FEV1 usually is measured in litre.

US 6,179,784 discloses a device and a method for sampling and monitoring a variety of respiratory profile parameters, for example the respiratory flow rate. The method detects the breath and may calculate various respirator parameters, such as the PEF or the FEV1.

Physicians and patients can gain several advantages by having access to accurate respiratory status data. A physician may evaluate the efficacy of the current medication regime. He may detect seasonal patterns, a rising, falling, personal best or trends of PEF and FEV1 values. There may be an assessment of airway stability over large blocks of time. The data may give information about compliance with a self management program, including daily peak flow monitoring and may provide a basis for an incentive system that physicians and/or parents can use to reward good compliance.

PEF and FEV1 are useful in detecting the early signs of airway instability and in evaluating the efficacy of medication regimens. For instance, a patient can take PEF samples before and after administering a bronchodilator and thus have a basis upon which to evaluate the drug's efficacy in treating that patient's acute asthma episodes.

A condition for the use of the respiratory status data are reliable measurements. The reliability depends on the measurement device but also on the patient's measuring technique and skill.

A consistent performance with a reduced variability in peak flow readings is needed, what can for example be achieved by a proper peak flow technique.

Most peak flow meter do not give any information on whether the patient has expired correctly when using the meter or whether the patient has produced a significant peak flow value. For example an improper expiration might have two maximum flow values. A standard device would take the higher measurement of the two values and display or use it as PEF, although the value is not representative for the physical state of the patient. Therefore it is sometimes difficult for a physician to make proper follow up diagnosis and a treatment plan adjustment according to this lack of information provided by the patient self- measurement at home.

Even if the patients are trained by medical personal how to expire correctly they do not have any feed back as to whether they expire correctly and whether the PEF and FEV1 values they collect at home are a good representation of their condition.

To give the patient a feed back about his expiration technique US 7,211,049 proposes to record a first breathing state of a person and to detect a deviation form the recorded first breathing state in subsequent breaths.

The device helps to receive subsequent measurements with a constant performance which is particularly beneficial because the patient usually inhales and exhale into a breathing tube, therefore he does not breath normally and it is difficult for him to control the uniformity of his breathing technique.

Due to the deviation the following measurement may be accepted or not. But since the device does not show the deviation there is no educational or training effect for the patient and he does not get support for correcting his breathing technique.

A control of expiration technique is also provided by a peak flow meter windmill trainer.

By attaching a windmill trainer to the meter, and blowing through the mouthpiece, exhaust air spins the sails. If the windmill is placed close to the mouthpiece, it is easy to make the sails spin but by moving the windmill further and further away from the mouthpiece, the person has to blow harder thus training the lungs.

Obviously, this is the very essence of good technique, and is particularly suitable for children below 10 years of age, and others who need help with their peak flow manoeuvre.

However, the windmill trainer does not give a quantitative feedback about the expiration technique and does for example not indicate multiple peaks of the expiration flow.

It is the object of the invention to overcome the drawbacks of the state of the art, in particular to provide a measuring system, a computer program, a set of a measuring device and a computer program and a method for operating the measuring system for controlling and for improving the breathing technique of a patient, which results in reliable expiration data.

In accordance with the present invention a measuring system for electronically measuring the volume flow expired and/or inspired by a user is presented. The measuring system comprises a measuring device with a measuring unit for continuously monitoring the flow of air streaming through a section of the device when the user is expiring and/or inspiring, thereby generating data of an expiration and/or inspiration volume flow profile. The measuring unit can be part of a flow meter or any other measuring device for measuring respiratory values as a spirometer or a pneumotachometer.

The volume flow profile illustrates the flow values versus time, for example as a curve of flow values versus a time scale.

The flow meter contains a first memory section for storing data of a reference expiration and/or inspiration volume flow profile and at least one second memory section for storing data of a measured expiration and/or inspiration volume flow profile. The measuring system further comprises display means for displaying flow profiles. The measuring system is constructed for displaying a representation of the reference flow profile and the measured flow profile, which allows a comparison of the profiles. The relationship between the flow profiles can be shown by simultaneously displaying the reference flow profile and the measured flow profile, preferably in parallel.

The reference flow profile may for example be displayed above or below the measured flow profile, it may be displayed in the background, the profiles may be displayed consecutively or alternately or in any other presentation which is suitable for comparison.

Alternatively a representation of data derived from the reference flow profile and the measured flow profile may be displayed, for example the difference between the flow profiles.

As a further option only parts, particularly relevant parts, of the flow profiles may be displayed.

The representation of the flow profiles provides to identify a relationship between the profiles and the possibility directly to compare the flow profiles.

Whereas most respiratory measurement systems only display some characteristic values, the system according to the present invention displays the complete necessary information, for example the complete expiration and/or inspiration flow profile together with a reference flow profile.

The display means are preferably suitable for displaying an expiration and/or inspiration flow profile being represented by a curve of the acquired volume data over the measurement time.

Display means for displaying alternative representations as a colour code representation, a histogram, a bar chart or any other representation may be also be used.

Depending on which part of the volume data shall be displayed the measuring system also comprises means for timely synchronising the measured profiles and the reference profile, for example with respect to the beginning of expiration and/or inspiration.

The displaying of a representation of the reference and the measured profile, which allows comparison of the profiles, for example by displaying of both the just measured flow profile and a reference profile, provides the opportunity for finding deviations and for deciding whether the measured inspiration and/or expiration flow is appropriate for determining significant respiratory parameters.

The user may immediately control his flow profile and check whether the data are useful for characterising the physical state.

In one embodiment of the invention display means are formed as a display integrated into the measurement device. A measuring device such as a flow meter and display means are integrated in one device, which may be used with all functions without connecting it to a separate device. Integrative devices of this kind are particularly advantageous for home-measurements and daily self control.

According to an alternative embodiment display means are formed as a separate display unit connected to or connectable to the measuring device.

The measuring device without display may be used for example as a conventional flow meter, but together with the display means it provides the possibility for training and improving the respiration technique of the patient.

The whole measurement system may be in possession of a physician or a clinic. The patient may be trained with the help of the display means by a qualified person. He may take home the flow measuring device for self management monitoring of his respiratory parameters.

The display means can be part of a PC-system. The flow meter may be connected to the PC, for example by an USB port, for transferring the data to the PC and displaying the flow profiles on the computer monitor.

In a preferable embodiment the measuring device comprises an interface for data transfer, particularly for importing data of a reference profile or for exporting measured data.

Advantageously the flow meter may be connectable, for example by an USB port, to an peripheral device, for example a PC. By input means of the peripheral device, for example a keyboard, a keypad, a touch screen or the like, one of the measured profiles may be selected and be stored as a reference profile on the flow meter. The data of the reference profile may also be taken from a databank or a look up table.

The measuring device comprises means for measuring the air flow. The measuring device may use any of the measuring methods known form the state of the art.

Preferably the measuring device comprises means for measuring the air flow by infrared measurement. The air flows through a swirl plate, which drives a rotating vane. The faster the air flow, the faster the vane rotates. An IR beam is cut off when the vane is on its way. By counting the cut frequency the rotating speed can be determined and the air flow can be calculated on the basis of the speed.

The measuring device may also display respiratory parameters and eventual warnings comparable to conventional peak flow meters.

In an advantageous embodiment of the invention the system comprises means for selecting a measured flow profile and/or storing it as a reference flow profile. The system may comprise input means, for example push buttons, for allowing the user to access and to select a certain measured flow profile.

In a further preferable measuring embodiment of the invention the system comprises means for automatically comparing a measured flow profile and a reference flow profile. The system may generate an output signal depending on the outcome of the comparison.

If for example the difference between the profiles is meant to be significant, the system may ask the user to take an other measurement.

According to a further aspect of the invention a software program is provided. The software program comprises program code means for performing the following steps when said program is run on a microprocessor, preferably to use in combination with a measuring system described above.

In a first step data measured by a measuring device measuring the volume expired and/or inspired by a user, are received.

The received data may be raw data or may correspond to volume flow values.

In a further step an expiration and/or inspiration flow profile are generated. The respective data are stored in a memory storage.

The flow profile may be stored by storing data being related to the volume flow values or by storing data being a representation of the volume flow values, which is suitable for displaying.

Finally the software program comprises program code means for performing the step of displaying a representation of at least two profiles, which allows a comparison of the profiles, on a display being connected with the microprocessor. In particular at least two profiles are displayed in parallel.

Displaying a representation of at least two profiles which allows a comparison of the profiles provides the opportunity to realize both profiles at the same time. The profiles may be displayed simultaneously, consecutively or alternating.

Preferably the data of the flow profiles are represented by a curve of volume flow over time, but alternative representations as a code representation, a histogram, a bar chart or any other representation may be used, too.

Only one expiration and/or inspiration cycle starting from the beginning of the expiration and/or inspiration is stored and/or displayed. Advantageously the program steps comprise the step of synchronising the displayed flow profiles for example with respect to the beginning of the inspiration or expiration.

By displaying the profiles in parallel the profiles can be compared easily. It is possible to compare data of successive measurements or data of a measured profile and a reference profile.

The software program provides a beneficial supplement for conventional software programs which display respiratory parameters in graphic or numeric form. Those programs are not suitable for comparing separate respiratory volume cycles.

The user gets an immediate and intuitive feed back about his respiratory technique compared with a previous measurement or a reference measurement.

In a preferred embodiment of the invention the software program comprises further program code means for performing the step of uploading a reference profile from a memory and displaying it as one of the profiles. The reference profile is chosen to represent a favourable flow profile with respect to the person of whom measurements are taken.

As a further step the software program preferably comprises further program code means for performing the step of selecting an expiration and/or inspiration flow profile as a reference profile and storing it in a memory.

One measurement which was found to result in an exemplary profile for determining reasonable respiratory parameters may be chosen by the user or by a skilled person, for example a physician, as data for a reference profile. This profile forms the guideline for further measurements. When it is displayed together with a just measured profile it serves as a standard according to which the patient or the physician may decide about the reliability of the measurement.

Alternatively or additionally the software program comprises further program code means for performing the step of receiving reference data by an input device and storing it on a memory.

The reference data may be chosen from a look up table, wherein the data depend for example on age, weight or height of the patient. The reference data may also have been generated previously in a separate process and/or on a different device.

The reference profile may for example be created by averaging a number of measured profiles.

In an advantageous embodiment of the invention the software program comprises further program code means for performing the step of determining and displaying or the steps of determining and displaying and storing conventional respiratory parameters, such as PEF and/or FEV1 values, basing on the received data.

Favourably the software program comprises further program code means for performing the step of creating an output signal depending on a difference of the reference profile and a measured expiration and/or inspiration flow profile. If the deviation of the measured profile with respect to the reference profile is greater than a predefined threshold, the measured data are not suitable for being the basis for an analysis of the physical state. In this case the program provides a warning, such that the measurement can be repeated.

For this and other reasons the software program may comprise further program code means for performing the step of comparing the measured profile with the reference profile.

For deciding about the optimal respiration technique it is not necessary to compare the absolute values of the inspiration and/or expiration flow. It may be sufficient to observe the progress and the characteristics of the flow curve over time. For example the expiration curve should not have two maxima.

In one embodiment of the invention the program is to be run on a peripheral device, such a PC or laptop, a Personal Digital Assistant, a mobile phone or a medical monitoring system.

The program may be used in a medical practice or in a clinic, to train the patient's respiratory technique by a skilled person. The skilled person may have the measured and the reference data at his disposal for giving recommendations with respect to respiratory techniques and further treatment of a patient.

The program may also be used by a patient on his own PC in his used environment. The data may be exchanged and controlled by an internet connection with the device of a skilled person.

In an alternative embodiment the program is to be run on a microprocessor being part of a measuring system or a measuring device, such as a flow meter. The device can then be used with all features independently of a separate device.

According to a further aspect of the invention a set is provided, comprising a measuring device for measuring the volume expired and/or inspired by a user with a data communication interface to a peripheral device, particularly a PC, and a software program implementable on the peripheral device, as described above.

The measuring device may comprise display means and thereby the measuring device may be an integrated measuring system as described above.

The user may use the measurement device as a conventional peak flow meter and nevertheless he or a skilled person gets the opportunity to control and to improve the respiratory technique and the reliability of the measured data.

According to a further aspect of the invention a method for operating a measuring system, preferably as described above, is provided. The method comprises the steps of continuously monitoring the volume flowing through a section of a measuring device when the user is expiring and/or inspiring, thereby generating data of an expiration and/or inspiration volume flow profile. The method further comprises the steps of storing data of at least one measured flow profile in at least one memory section and of displaying a representation of the reference flow profile and the at least one measured profile on display means, which allows comparison of the profiles, in particular simultaneously displaying the reference flow profile and the at least one measured profile, preferably in parallel.

The reference flow profile may be downloaded from a web data base, where it has been uploaded by a skilled person.

Preferably the method further comprises the step of storing a reference flow profile in a first memory section. The memory section may be part of or may be connectable to the measuring system.

The reference profile may be received from an input device, may be chosen from a data base or a look up table or may have been generated in a separate process.

Alternatively the method may comprise as a first step the step of selecting a reference flow profile from the measured volume data.

Typically a skilled person will control the measured data and select one profile as a reference profile to store on a memory and to be available for further measurements as a standard.

Further benefits and advantages of the present invention will be described on the description and with reference to the following drawings.

### Brief description of the drawings:

- Figure 1: is a perspective view of an integrated measuring system;
- Figure 2: is a block diagram of the measuring system;
- Figure 3: is a flow chart describing the method for operating the measuring system;
- Figure 4: shows three examples of expiration curves;

- Figure 5: shows two examples of two flow profiles which are displayed together with a reference profile.

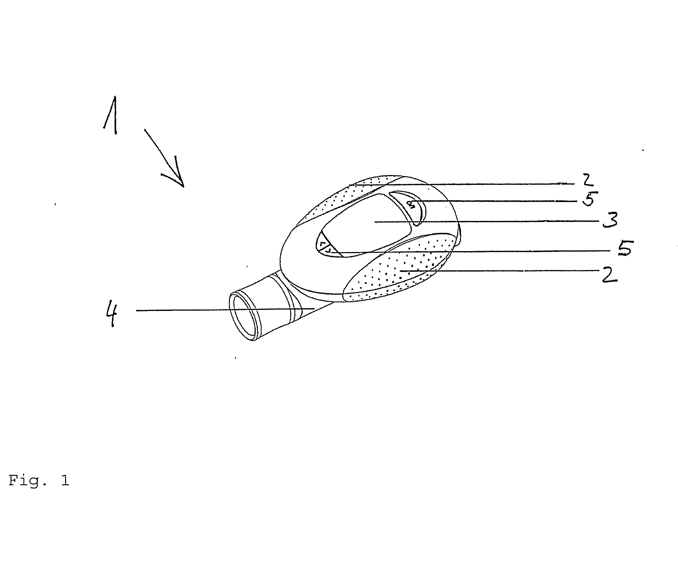

Figure 1 is a perspective view of an integrated measuring system 1. The measuring system 1 comprises a flow meter 2 and display means 3. The display means 3 are integrated and part of the measuring device 2.

In the present example the display means 3 is an LCD monitor.

The flow meter 2 comprises a mouth piece 4 for blowing air into a measuring unit not shown in detail this figure.

The flow meter 2 further comprises operating buttons 5 for switching on and off the system 1, for selecting operation modes, for storing measured data or for other method steps provided by the measuring system 1.

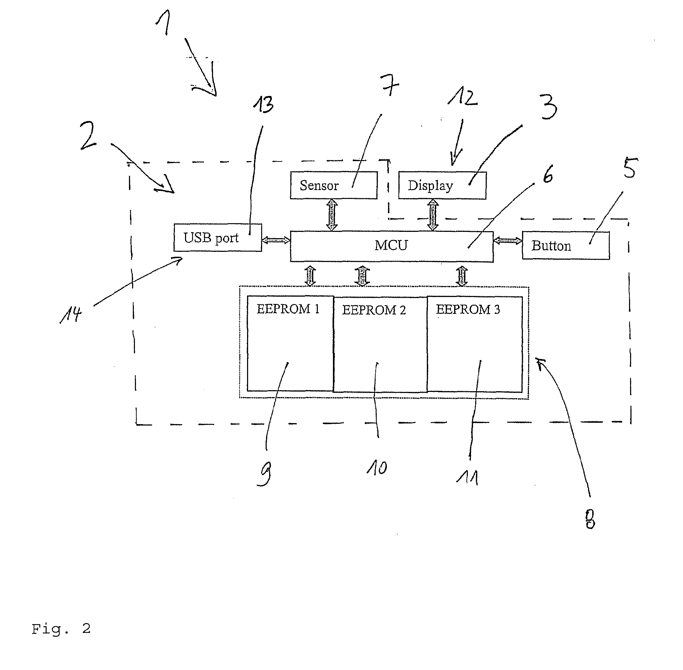

Figure 2 is a block diagram of an example of the measuring system 1.

The measuring device 1 comprises a measuring device 2 and display means 3. The central part of measuring device 2 is a microcontroller unit 6 being in connection with a measuring unit 7 for monitoring the respiratory volume.

The measuring unit 7 may comprise means for infrared measurement of flow values not explicitly shown in the figures. The air flows through a swirl plate, which drives a rotating vane. The faster the air flow, the faster the vane rotates. An IR beam is cut off when the vane is on its way. By counting the cut frequency the rotating speed can be determined and the air flow can be calculated on the basis of the speed.

Alternatively, the flow may be measured by other methods: it is e.g. possible to use two sensors arranged along the path of the air on two sides of a reduction of the cross-section of the flow path. By detecting the difference in pressure and temperature, the flow volume may be calculated on the basis of pressure and temperature measurements made at the place before and after the reduction of cross-section. Such difference measurement is known per se. It is also possible to use a resistance strain gauge arranged perpendicularly to the direction of the flow. Depending on the flow, the sensor will be curved more or less and thereby will allow to calculate the flow volume.

The measurement and the analysis of the data may be affected and controlled by buttons 5 pushed by the user.

The flow meter 2 comprises an EEPROM 8 with a first memory section 9 for storing data of an optimal expiration profile, a second memory section 10 for storing data of a measured expiration profile and a third memory section 11 for storing PEF and/or FEV1 data. Alternatively, a flash memory can be used for storage of the data.

The graphs and/or data or any representation of the data as well as the operating mode may be displayed on display means 3. The display means 3 may be integrated into and be part of the flow meter 2 or the display means 3 is formed as a separate display unit 12 and may be connected to the flow meter 2.

The microcontroller unit is connected to an USB port 13, which is part of an interface 14. The interface 14 may be used for transferring data from and to the flow meter 2. For example a reference flow profile may be imported via the interface 14.

Alternatively, instead of a USB port, a serial port (RS232) or wireless interfaces such as infrared, blue tooth or RF interfaces may be used.

The microcontroller unit is typically a standard 8bit controller.

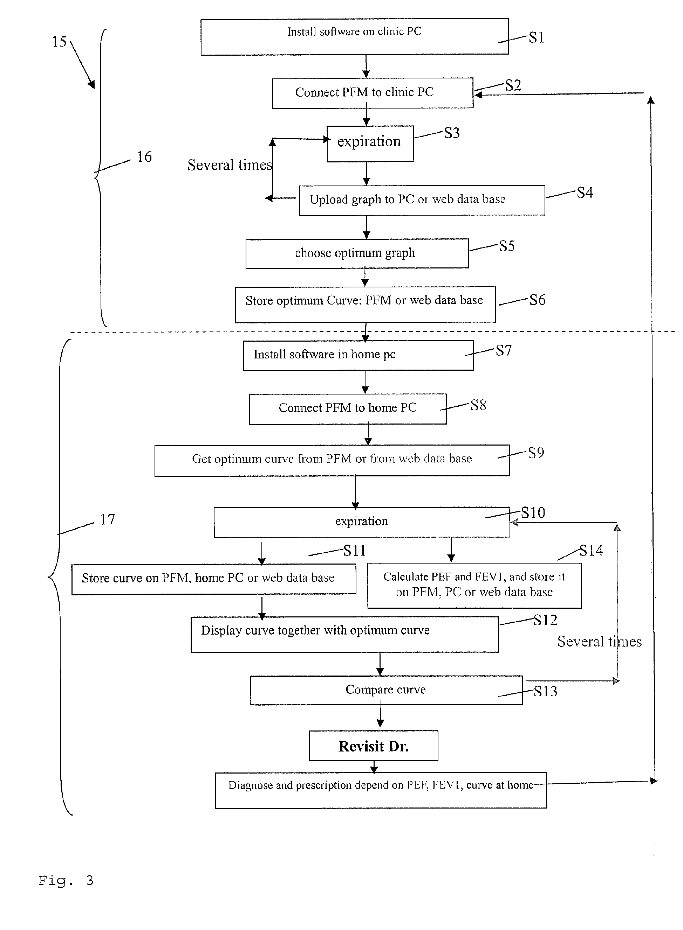

Figure 3 is a flow chart 15 describing the method for operating the measuring system 1.

The first part 16 of the method usually takes place under responsibility of a skilled person, for example in a medical practice or in a clinic. During this first part a patient is coached by the skilled person with respect to a suitable respiratory technique.

In a first step S1 a software program with code means for performing the method steps is installed in a clinic PC. A personal peak flow meter PFM of a patient in a second step S2 is connected to the clinic PC. The patient may then expire into his own PFM (step 3 S3) and a graph showing the expiration volume over time is displayed on the display of the PC after uploading to the PC or to the web data base (step 4 S4). The skilled person may decide whether the graph is a good representation of a suitable expiration flow profile. An expiration profile may be recorded several times to create a variety of samples for choosing an optimum graph in a fifth step S5. This optimum graph is stored as a reference flow profile either directly on a memory section of the PFM or on a web data base in sixth step S6.

The patient continues with the second part 17 of the method within a self management program at home. He therefore installs the software program on his own PC at home (step 7 S7) and connects the PFM to his home PC (Step 8 S8), uploads the reference flow profile either from a memory section of the PFM or from a web data base (step 9 S9).

At the latest when beginning the respiratory self control,the patient records his expiration volume profile (step 10 S10) which is stored in a memory section either of the PFM, in a memory section of the home PC, or on a web data base (step 11 S11). The data are transmitted to the PC and are displayed on display means of the PC together with the reference flow profile (step 12 S12). The patient may now compare the two profiles (step 13 S13) and he may decide whether the measured profile is similar to the reference profile and whether it is necessary to record another expiration flow profile.

Alternatively as a further step of the program the profiles are compared automatically. If the deviation of the measured profile is not within a predetermined value, the PC will generate an output signal which asks the patient to repeat the measurement.

Subsequent to expiration in step S10 and parallel to step S11, S12 and S13, in a further step S14 the PEF and FEV1 values may be determined form the measured volume.

These values may be stored on the PFM or a Web data base or may be transmitted to the clinic PC. Due to the values the physician will decide about the further therapy.

Alternatively all data may be stored directly on an integrated measurement system, for example on memory sections of a flow meter, such that a connection to a PC for data transfer is not necessary.

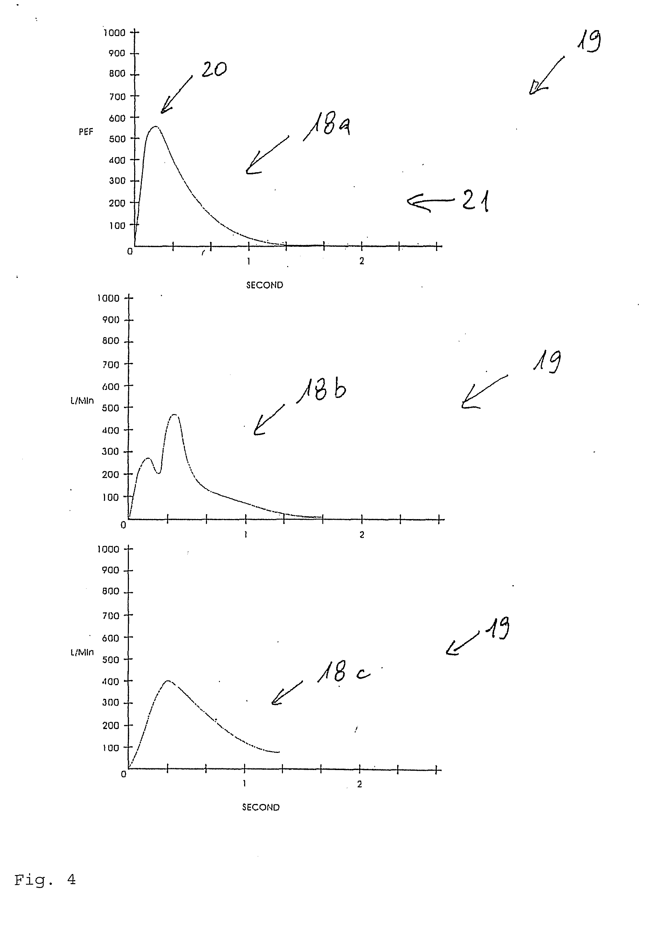

Figure 4 shows three examples of expiration curves 18a, 18b, 18c. These expiration profiles 19 are represented by a curve of flow values, measured in litre per minute over the time measure in seconds. The PEF value 20 is determined as the maximum value of the curve.

Expiration curves are recorded during the first part of the method as shown in figure 3. A skilled person may now choose the optimum graph and store it as a reference profile. In the example shown by figure 4 the first curve 18a would be chosen as reference profile 21. A curve with a profile of the second curve 18b is not suitable to determine a reasonable PEF value, because the curve has two maxima. The same holds for the third curve 18c due to the long declination which does not correspond to a forced respiration.

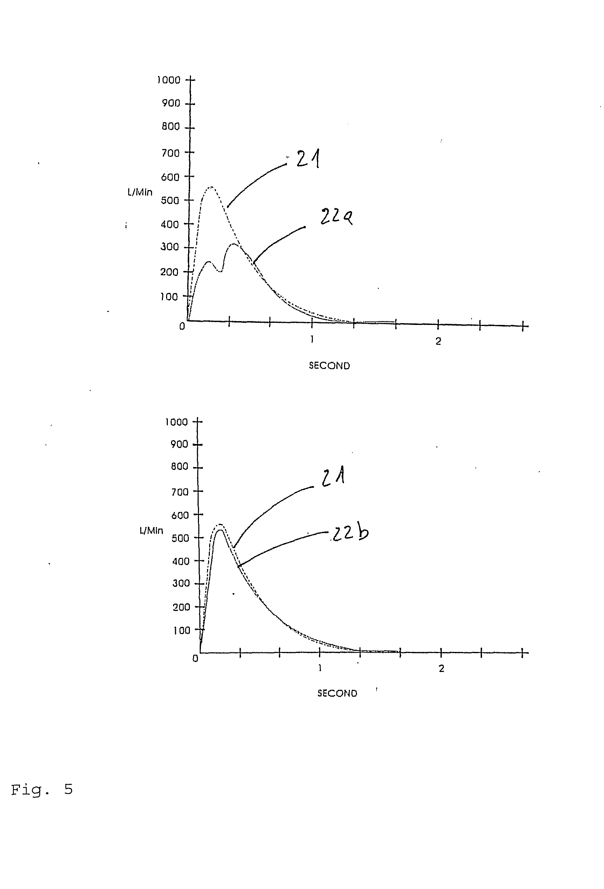

Figure 5 shows two examples of two measured flow profiles 22a, 22b which are displayed together with a reference profile 21. The reference profile 21 is presented as a background within the same scales as the measure profiles 22a, 22b, such that an immediate and intuitive comparison is possible.

In case a patient has recorded the first flow profile 22a he will immediately see, that the measured flow profile 22a is completely different from the reference profile 21 and he will repeat the measurement until he gets for example the second profile 22b.

## Claims

1. A measuring system for electronically measuring the volume flow expired and/or inspired by a user, the system comprising a measuring device, particularly a flow meter (2), with
- a measuring unit (7) for continuously monitoring the volume flow of air streaming through a section of the measuring device (2) when the user is expiring and/or inspiring, thereby generating data of an expiration and/or inspiration volume flow profile (19),
- a first memory section (9) for storing data of a reference expiration and/or inspiration volume flow profile (21) and
- at least a second memory section (10) for storing data of a measured expiration and/or inspiration volume flow profile (22a, 22b), the measuring system further comprising
- display means (3) for displaying flow profiles (19),
wherein the measuring system is construed for displaying a representation of the reference flow profile (21) and the measured flow profile (22a, 22b), which allows a comparison of the profiles, in particular simultaneously displaying the reference flow profile (21) and the measured flow profile (22a, 22b), preferably in parallel.

2. A measuring system according to claim 1, wherein the display means (3) are formed as a display integrated into the measuring device (2).

3. A measuring system according to claim 1 or 2, wherein the measuring system comprises display means (3) formed as a separate display unit connected to or connectable to the measuring device (2).

4. A measuring system according to one of claims 1 to 3, wherein the measuring device comprises an interface (14) for data transfer, particularly for importing data of a reference flow profile (21) or exporting data of measured volume flow profiles (22a, 22b).

5. A measuring system according to one of claims 1 to 4, wherein the measuring device comprises means for measuring the air flow by infrared measurement.

6. A measuring system according to one of claims 1 to 5, wherein the measuring systems comprises means for selecting a measured flow profile (18a, 18b, 18c) and/or storing it as a reference flow profile (21).

7. A measuring system according to one of claims 1 to 6, wherein the measuring system comprises means for automatically comparing a measured flow profile (22a, 22b) and a reference flow profile (21).

8. A software program comprising program code means for performing the following steps when said program is run on a microprocessor, preferably to use in combination with a measuring system according to claims 1-7,
- receiving data measured by a measuring device (2), said data representing the volume flow expired and/or inspired by a user;
- generating an expiration and/or inspiration flow profile (19, 21; 22a, 22b) on the basis of said data;
- storing said profile on a memory storage (9, 10);
wherein the software program comprises further program code means for performing the step of displaying a representation of at least two flow profiles (19, 21; 22a, 22b), which allows a comparison of the profiles, on display means (3) being connected with the microprocessor (6), in particular displaying at least two profiles in parallel.

9. A software program according to claim 8 comprising further program code means for performing the step of uploading a reference profile (21) from a memory (9) and displaying as one of the profiles.

10. A software program according to claim 8 or 9 comprising further program code means for performing the step of selecting an expiration and/or inspiration flow profile (19) as a reference flow profile (21) and storing it in a memory (8).

11. A software program according to claim 8 to 10 comprising further program code means for performing the step of receiving reference data by an input device and storing it in a memory (8).

12. A software program according to claim 8 to 11 comprising further program code means for performing the step of determining and displaying or for determining and displaying and storing PEF and/or FEV1 values basing on the received data.

13. A software program according to claim 8 to 12 comprising further program code means for performing the step of creating an output signal depending on a difference of the reference profile and a measured expiration and/or inspiration flow profile.

14. A set of a measuring device for measuring the volume flow expired and/or inspired by a user with a data communication interface to a peripheral device, and a software program implementable on the peripheral device according to one of claims 8 to 13.

15. A method for operating a measuring system, preferably according to claims 1-7, comprising the steps of
- continuously monitoring the flow of air streaming through a section of the device when the user is expiring and/or inspiring, thereby generating data of an expiration and/or inspiration volume flow profile (22a, 22b),
- storing data of at least one measured volume flow profile in at least one memory section and
- displaying a representation of a reference flow profile (21) and the at least one measured flow profile (22a, 22b), which allows a comparison of the profiles (21, 22a, 22b), in particular simultaneously displaying the reference flow profile (21) and the measured flow profile (22a, 22b), preferably in parallel.

16. A method for operating a measuring system according to claim 15, the method further comprising the step of storing a reference flow profile (21) in a first memory section (9).

17. A method for operating a measuring system according to claim 15, the method further comprising the step of selecting a reference flow profile (21) from measured volume data.
